Europäisches Patentamt

European Patent Office    (11) Publication number: **0 311 108**

Office européen des brevets    **A2**

(19)

## (12) EUROPEAN PATENT APPLICATION

(21) Application number: **88116668.0**    (51) Int. Cl.⁴: **C07H 19/173 , A61K 31/70**

(22) Date of filing: **07.10.88**

(30) Priority: **08.10.87 US 106137**

(43) Date of publication of application:
**12.04.89 Bulletin  89/15**

(84) Designated Contracting States:
**AT BE CH DE ES FR GB GR IT LI LU NL SE**

(71) Applicant: **Stichting REGA V.Z.W.**
**Minderbroedersstraat 10**
**B-3000 Leuven(BE)**

(72) Inventor: **Balzarini, Jan Maria René**
**Prospor Poulletlaan 10/9**
**B-3000 Leuven(BE)**
Inventor: **Herdewijn, Piet**
**Diestsesteenweg 232**
**B-3200 Kessel-Lo(BE)**
Inventor: **De Clercq, Erik Désiré A.**
**Paul Lebrunstraat 35/7**
**B-3000 Leuven(BE)**
Inventor: **Robbins, Morris J.**
**1831 North 2050 West**
**Provo Utah 84604(US)**

(74) Representative: **Prins, Hendrik Willem et al**
**Octrooibureau Arnold & Siedsma**
**Sweelinckplein, 1**
**NL-2517 GK The Hague(NL)**

(54) Anti-HIV active 3'-azido-2,6-diaminopurine-2',3'-dideoxyriboside.

(57) The novel sugar-modified 2,6-diaminopurine-2',3'-dideoxynucleoside: 3'-azido-2,6-diaminopurine 2',3'-dideoxyriboside selectively inhibits the cytopathogenicity and replication of retroviruses (i.e. human immunodeficiency virus).

FIG.2

EP 0 311 108 A2

# Anti-HIV active 3'-azido-2,6-diaminopurine-2',3'-dideoxyriboside

This invention relates to a novel sugar-modified 2,6-diaminopurine-2',3'-dideoxynucleoside, to its application as novel therapeutic agent as well as to its preparation.

A series of purine and pyrimidine nucleoside analogues with the sugar moiety in the 2',3'-dideoxyribose configuration has proven successful in the inhibition of the replication of human immunodeficiency virus (HIV) in vitro (Balzarini, J. et al, Biochem. Biophys. Res. Commun. 140:735-742 (1986a); Balzarini, J. et al, Mol. Pharmacol. 32:162-167 (1987a); Baba, M. et al, Biochem. Biophys. Res. Commun. 142:128-134 (1987a)). The most potent and selective inhibitors so far are 2',3'-dideoxycytidine (ddCyd), 2',3'-dideoxythymidine (ddThd) (Baba, M. et al (1987)), the 2',3'-unsaturated pyrimidine 2',3'-dideoxynucleoside analogues derived thereof (ddddCyd and ddddThd) (Balzarini, J. et al (1987a)), 5-fluoro-ddCyd, 3'-fluoro-ddThd (FddThd) (Herdewijn, P. et al, J. Med. Chem. 30:1270-1278 (1987a)), 2',3'-dideoxyadenosine (ddAdo), 3'-azido-ddAdo (AzddAdo) (Herdewijn, P. et al, J. Med. Chem., (1987b)), 2',3'-dideoxyguanosine (ddGuo), and 2',3'-dideoxyinosine (ddIno). Recently, we reported on 3'-azido-ddGuo (AzddGuo) (Baba, M. et al, Biochem. Biophys. Res. Commun. 145:1080-1086 (1987b)) and 2,6-diaminopurine 2',3'-dideoxyriboside (ddDAPR) (Balzarini, J. et al, Biochem. Biophys. Res. Commun. 145:269-276 (1987b)) as potential new anti-AIDS drugs. AzddGuo completely inhibits the HIV-induced cytopathogenicity and viral antigen expression in MT4 cells at 5 μM, and was more selective than ddGuo in its anti-HIV activity. ddDAPR is, like ddAdo, a potent and selective inhibitor of HIV in vitro, and inhibits HIV-replication in MT4 cells at a 50% effective dose (ED$_{50}$) of 2.5-3.6 μM.

During further research for new potential anti-retroviral drugs we synthesized new purine 2',3'-dideoxyribosides with modifications in the sugar moiety.

We found that 3'-azido-2,6-diaminopurine 2',3'-dideoxyriboside (AzddDAPR), is a potent and selective anti-retrovirus agent in vitro. Its potency and selectivity as anti-HIV agent are higher than recorded for ddDAPR.

Hereafter, AzddDAPR will be compared with several other purine 2',3'-dideoxyribosides.

## MATERIALS AND METHODS

2,6-Diamino-9-(2,3-dideoxy-β-D-glycero-pentofuranosyl)purine (2,6-diaminopurine 2',3'-dideoxyriboside, ddDAPR) was synthesized according to a procedure used for conversion of adenosine into its 2',3'-unsaturated derivative 2',3'-didehydro-2',3'-dideoxyadenosine (ddddAdo) and subsequent hydrogenation of ddddAdo to give ddAdo (McCarthy Jr., et al, J. Am. Chem. Soc. 88:1549-1553 (1966); Robins , M.J. et al, Tetrahedron Lett. 25:367-370 (1984); Hanske, F. et al, Tetrahedron Lett. 26:4295-4298 (1985)).

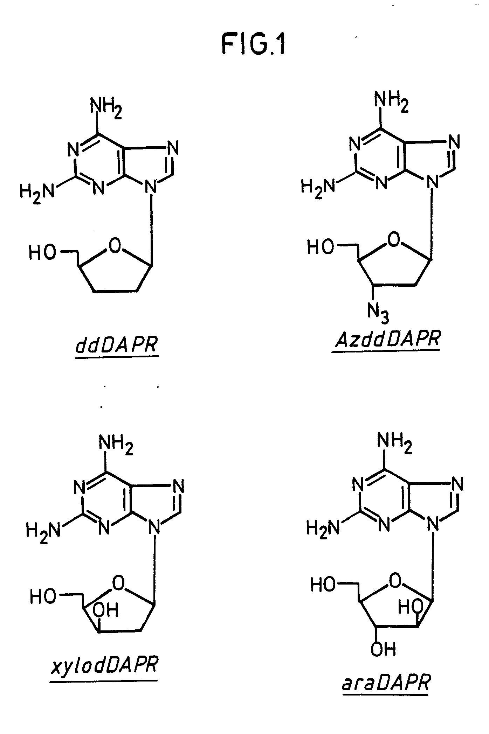

3'-Azido-ddDAPR (AzddDAPR) was prepared in very low yield by the chemical transfer glycosylation procedure of Imazawa and Eckstein using 1-(3-azido-2,3-dideoxy-β-D-erythro-pentofuranosyl)thymine (AZT) and 2,6-diacetamidopurine (J. Davoll et al, J. Am. Chem. Soc. 73:1650-1655 (1951)). Work-up was followed by deprotection with methanolic aqueous ammonia and purification on columns of Dowex 1X2 (OH$^-$) followed by silica gel. The 2,6-diamino-9-(3-azido-2,3-dideoxy-β-D-erythro-pentofuranosyl)purine (AzddDAPR) had mp 239-241 °C (dec.); MS m/z 291.1195 (M$^+$ [C$_{10}$H$_{13}$N$_9$O$_2$] = 291.1192; $^1$H NMR (Me$_2$SO-d$_6$, Me$_4$Si) δ6.15 ("t", J$_1$'-2'$_2$" = 6.5 Hz, 1, H1'). AzddDAPR was also synthesized from 2,6-diamino-9-(3-O-2-deoxy-3-O-mesyl-β-D-threo-pentofuranosyl)2,6-diaminopurine with lithium azide in dimethylformamide. The synthesis of 9-β-D-arabinofuranosyl 2,6-diaminopurine (araDAPR) has been described previously (Hanske F. et al, Tetrahedron Letters 40: 125-135 (1984)). The structural formulae of ddDAPR, AzddDAPR, XylodDAPR and araDAPR are depicted in Fig. 1. 2',3'-Dideoxyguanosine (ddGuo) was obtained from Pharmacia PL-Biochemicals (Uppsala, Sweden). 3'-Azido-2',3'-dideoxyguanosine (AzddGuo) was synthesized by M. Imazawa and F. Eckstein (Imazawa, M. et al, J. Org. Chem. 43:3044-3048 (1978)). All reagents used were of the highest quality available.

Therapeutic compositions, containing AzddDAPR as an active ingredient for treating AIDS, AIDS-related diseases and other retroviral diseases, including hepatitis B, in human practice may take the form of powders, suspensions, solutions, sprays, emulsions, unguents or creams and may be used for local application, for intranasal, rectal, vaginal and also for oral or parenteral (intravenous, intradermal, intramuscular, intrathecal etc.) administration. Such compositions may be prepared by combining (e.g. mixing, dissolving etc.) the active compound with pharmaceutically acceptable excipients of neutral character (such as aqueous or non-aqueous solvents, stabilizers, emulsifiers, detergents, additives), and further, if necessary with dyes and aromatizers. The concentration of the active ingredient in the therapeutic composition may vary widely between 0.1% and 100%, dependent on the mode of administration. Further, the dose of the active ingredient to be administered may vary between 0.1 mg and 100 mg per kg of body weight.

The pharmaceutical properties of AzddDAPR concerning this anti-retroviral action, especially HIV, are documented by the following examples which should not be read in a restrictive sense.

In the examples reference is made to the attached drawings in which:

Fig. 1 comprises structural formulae of ddDAPR, AzddDAPR, XylodDAPR and araDAPR;

Fig. 2 is a bar representation of the test results on the inhibition of the cytopathogenic effect of HIV in human T4 lymphocyte MT4 cells by ddDAPR and AzddDAPR. The viability of the cells was measured by trypan blue exclusion after an incubation period of 5 days at 37°C.

## CELLS

The cultivation of Raji, Molt/4F, CEM, H9, HUT-78 and MT4 cells has been described in Balzarini, J, et al, Biochem. Biophys. Res. Commun. 136:64-71 (1986b) and Pauwels, R. et al, J. Virol. Methods, 16:171-185 (1987).

## VIRUSES

HIV was obtained from the culture supernatant of a H9 cell line persistently infected with HTLV-III$_B$. Moloney murine sarcoma virus (MSV) was prepared from tumors induced by in vivo infection of 2-3 day old NMRI mice.

## ANTIVIRAL ASSAYS

For transformation of C3H mouse embryo cells by Moloney murine sarcoma virus (MSV), confluent monolayers of C3H cells in 1 ml wells of Tissue Culture Cluster plates were infected with 150 foci-forming units of MSV during 90 min, whereafter medium was replaced by 1 ml fresh culture medium containing different concentrations of test compound. After 6-7 days incubation at 37°C, transformation of the cell cultures was monitored microscopically.

For the determination of the cytopathic effect of HIV in human T-lymphocyte MT4 cells, MT4 cells, subcultured one day before the start of the experiment, were adjusted at $5 \times 10^5$ cells/ml and infected with HIV (HTLV-III$_B$) at 100 CCID$_{50}$/ml. Then, 100 $\mu$l of the infected cell suspension was brought into wells of a microtiter tray containing 100 $\mu$l of various dilutions of the test compounds. After 5 days incubation at 37°C, the number of viable cells was recorded microscopically in a hematocytometer by trypan blue exclusion.

The inhibitory effects of the test compounds on HIV antigen expression in infected H9 or HUT-78 cells were determined at day 8 and 14, respectively after infection, by indirect immunofluorescence and laser flow cytofluorography using a polyclonal antibody as probe, as previously described (Pauwels, R. et al, (1987)).

## ANTI-RETROVIRAL EFFECT

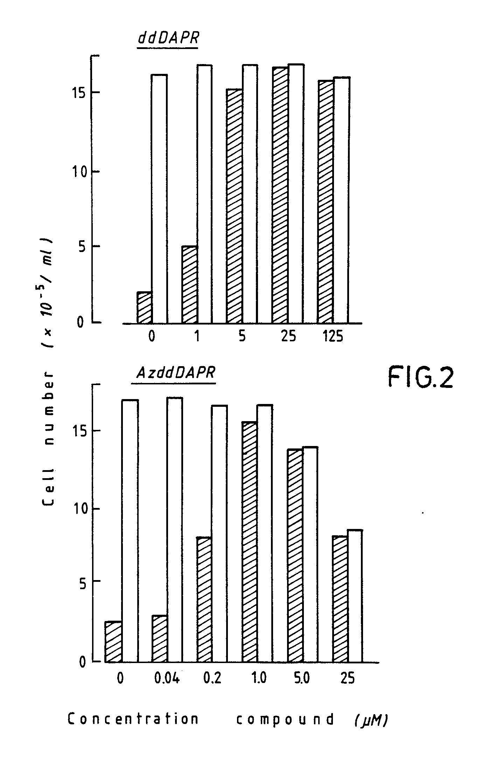

A series of novel 2,6-diaminopurine 2′,3′-dideoxynucleoside analogues in which the 2′,3′-dideoxyribose moiety is substituted by a 3′-azido group, or replaced by arabinose or 2′-deoxyxylose were evaluated for their effect on the transformation of C3H mouse embryo cells by Moloney murine sarcoma virus (MSV) (Table 1) and the cytopathogenicity induced by HIV in human T4 lymphocyte MT4 cells (Table 1, Fig. 2). AzddDAPR selectively inhibited HIV-induced cytopathogenicity in MT4 cells. AzddDAPR proved considerably more effective as the parent compound ddDAPR in inhibiting HIV infectivity but was also slightly more toxic to MT4 cells. Its selectivity index (SI) (ratio of 50% cell toxic dose to 50% antivirally effective dose) was somewhat higher (157) than recorded for ddDAPR (106) (Table 1). The 9-$\beta$-D-arabinoside derivative and 2′-deoxy-9-$\beta$-D-xyloside derivative of ddDAPR are devoid of any anti-HIV activity. AzddDAPR inhibited cell transformation by MSV at a concentration as low as 1.0 $\mu$M, that is at a 19 fold lower dose than required for ddDAPR. XylodDAPR and araDAPR have no anti-MSV effect at all at 400 $\mu$M.

To confirm our anti-HIV data obtained in the MT4 cell model, we also evaluated this 3'-substituted ddDAPR derivative in two other human T4 lymphocyte cell lines (H9 and HUT-78) for its inhibitory effect on viral antigen expression (data not shown). In these experiments, AzddDAPR achieves 50% inhibition of HIV-induced antigen expression at a concentration range of 0.09 - 0.5 $\mu$M. This concentration range is lower than that required to inhibit HIV-induced cytopathogenicity in MT4 cells.

## CYTOSTATIC AND ANTIMETABOLIC EFFECTS

The cytostatic effects of the compounds listed in Table 1 were examined against several human B-lymphoblast Raji, T-lymphoblast Molt/4F and T-lymphocyte CEM, H9 and MT4 cell lines (Table 2). None of the compounds evaluated showed severe cytostatic effects against any of the cell lines tested. In no case is the $ID_{50}$ of the compounds for tumor cell proliferation lower than 60 $\mu$M. XylodDAPR and araDAPR are devoid of any anticellular activity, even when tested at a concentration as high as 1000 $\mu$M.

Cellular DNA synthesis as monitored by the incorporation of [methyl-$^3$H]dThd into DNA of MT4 cells is not significantly affected by the test compounds at 200 $\mu$M

## EFFECT OF 2'-DEOXYCOFORMYCIN ON THE ANTI-RETROVIRAL AND CYTOSTATIC EFFECTS OF ddDAPR, AND AzddDAPR

The effect of preincubation of the cells by 10 $\mu$M 2'-deoxycoformycin (DCF) on the anti-HIV and anti-MSV activity, and the cytostatic action of ddDAPR and AzddDAPR has been evaluated (Table 3). Pretreatment of MT4 or C3H cells with DCF resulted in a significantly decreased anti-retroviral effect of ddDAPR. Also, pretreatment of Molt/4F, CEM and MT4 cells by DCF decreases considerably the cytostatic action of this compound (table 4). In contrast, the anti-HIV and anti-MSV activity of AzddDAPR as well as its inhibitory effect on CEM, Molt/4F and MT4 cell proliferation, is virtually unaffected in the presence of DCF.

## SUBSTRATE ACTIVITIES OF THE ddDAPR ANALOGUES FOR BEEF INTESTINE ADENOSINE DEAMINASE

Several ddDAPR analogues were examined for their susceptibility to deamination by beef intestine adenosine deaminase (ADA). The $K_m$ values of ADA for the natural substrates adenosine (Ado) and 2'-deoxyadenosine (dAdo) are 29 $\mu$M and 15 $\mu$M, respectively (Table 5, Balzarini, J. et al, Biochem. Biophys. Res. Commun. 145:277-283 (1987c)). ddDAPR shows a similar Km than Ado but a Vmax that is only 3% of that observed for Ado. AzddDAPR has a 2-fold lower $K_m$ (11 $\mu$M) and a 5-fold higher $V_{max}$ (40 $\mu$mole/mg protein/min) than ddDAPR. Thus, the ratio $V_{max}/K_m$ obtained for AzddDAPR proves 10-fold higher than that for ddDAPR, similar to ddAdo, and only 2 to 3-fold lower than for Ado (Table 5). Both araDAPR and XylodDAPR have similar $K_m$ and $V_{max}$ values, and their $V_{max}/K_m$ ratios are considerably lower than those recorded for ddDAPR and ddAdo.

The 3'-azido-substituted 2',3'-dideoxyriboside of 2,6-diaminopurine (AzddDAPR) proved significantly more effective than ddDAPR in inhibiting the cytopathogenicity of HIV in MT4 cells. AzddDAPR also has an in vitro chemotherapeutic index, higher than that of AzddGuo and ddGuo, the latter two compounds are known promising agents for the treatment of AIDS. AzddDAPR is also far more effective than the other purine 2',3'-dideoxyribosides listed in Table 1 as an anti-retroviral agent when evaluated in a murine model with Moloney murine sarcoma virus as the retroviral species.

Because of its prominent anti-HIV and anti-MSV activity and its relatively non-toxic properties in cell culture, AzddDAPR must be considered as a novel, potent and selective anti-retroviral agent, that should be further pursued for its efficacy in the treatment of retrovirus infections including AIDS, and hepatitis B.

The close similarity of the biological properties of ddDAPR and AzddDAPR to those of ddGuo and AzddGuo and the fact that ddDAPR and AzddDAPR are susceptible to deamination by beef intestine adenosine deaminase suggest that the 2,6-diaminopurine 2',3'-dideoxynucleosides may hypothetically exert their anti-retrovirus activity, at least in part, as prodrugs of ddGuo and AzddGuo, respectively.

However, when evaluated for their cytostatic and anti-retrovirus activity in combination with a potent inhibitor of adenosine deaminase (2'-deoxycoformycin, DCF), the biological effects of ddDAPR but not AzddDAPR are substantially decreased. These observations suggest that ddDAPR may act (in part, or completely) as prodrug of ddGuo while AzddDAPR might also exert its biological effects without obligatory prior conversion to its AzddGuo derivative.

Therefore, AzddDAPR may be considered as a drug that, irrespective of the conversion to its deaminated derivative AzddGuo, may still exert a potent anti-retrovirus activity and may be intracellularly active as either 2,6-diaminopurine or guanine nucleoside derivative. AzddDAPR might be advantageous over AzddGuo as an anti-retroviral agent. In deed, if both AzddDAPR and AzddGuo are converted to their corresponding 5'-triphosphate metabolites by different metabolic pathways, spontaneous deficiency of one of the enzymes involved in the pathway that activates AzddGuo will make HIV-infected target cells resistant to the anti-retrovirus effect of AzddGuo while deficiency of cells for such an enzyme will not induce resistance to AzddDAPR because this compound can still be activated through the other metabolic pathway.

The potent inhibitory effect of AzddDAPR on Murine C3Hcell transformation by MSV invitro, was also reflected by our in vivo experiments in which NMRI mice were infected at 3 days after birth with MSV.

Intramuscular inoculation of MSV in these mice resulted in tumor formation and mortality associated therewith. Daily treatment of the MSV-infected mice with AzddDAPR at 125 mg/kg/day dramatically increased the mean day of tumor formation from 5.3 to 11 days. 10% of the mice did not develop any tumor at all at day 28 of the experiment. Also, survival of the MSV-infected mice was significantly increased: average survival time was increased from 9.0 days for control group to 15.8 days for treated group. Moreover 30% survivors at day 22 of the experiment were recorded. When MSV-infected mice were treated with AzddDAPR at 25 mg/kg/day, the delay in tumor formation and survival of the mice was less pronounced but still significant. Mean day of tumor formation was increased from 5.3 days to 6.8 days, and the average survival time was increased from 9.01 days to 11.8 days.

In conclusion AzddDAPR is not only a potent inhibitor of retrovirus replicate in vitro, but it has also a pronounced activity against Moloney murine sarcoma virus (MSV) - induced tumor development in newborn NMRI mice in vivo.

Table 1

| Anti-retroviral and cytotoxic activity of sugar-modified 2,6-diamino-2',3'-dideoxynucleoside analogues | | | | | | |
|---|---|---|---|---|---|---|
| Compound | HIV-induced cytopathogenicity in MT4 cells | | | MSV-induced C3H cell transformation | | |
| | $ED_{50}(\mu M)$ | $CD_{50}(\mu M)$ | S.I. | $ED_{50}(\mu M)$ | $CD_{50}(\mu M)$ | S.I. |
| ddDAPR | 3.8 | 404 | 106 | 19 | $\geq 200$ | $\geq 11$ |
| AzddDAPR | 0.3 | 44 | 157 | 1.0 | $\geq 200$ | $> 200$ |
| XylodDAPR | $> 125$ | $> 625$ | - | $> 400$ | $> 400$ | - |
| araDAPR | $> 125$ | $> 625$ | - | $> 400$ | $> 400$ | - |

Table 2

| Cytostatic effect of sugar-modified 2,6-diaminopurine 2',3'-dideoxynucleoside analogues against human B- and T-cell lines | | | | | |
|---|---|---|---|---|---|
| Compound | Inhibitory dose-50 ($\mu M$) | | | | |
| | Raji | Molt/4F | CEM | H9 | MT4 |
| ddDAPR | 324 | 244 | 125 | 466 | 644 |
| AzddDAPR | 157 | 72 | 68 | 69 | 382 |
| XylodDAPR | $> 1000$ | $> 1000$ | $> 1000$ | $> 1000$ | $> 1000$ |
| araDAPR | $> 1000$ | $> 1000$ | $> 1000$ | $> 1000$ | $> 1000$ |

Table 3

| Effect of 2′-deoxycoformycin (DCF) on the anti-retrovirus activity of ddDAPR and AzddDAPR | | | |
|---|---|---|---|
| Compound | Presence of 10 μM DCF | Effective dose-50 (μM) | |
| | | HIV-induced cytopathogenicity in MT4 cells | MSV-induced C3H cell transformation |
| ddDAPR | - | 3.8 | 23 |
| | + | 13 | 84 |
| AzddDAPR | - | 0.28 | 1.3 |
| | + | 0.70 | 1.6 |

Table 4

| Effect of 2′-deoxycoformycin (DCF) on the cytostatic activity of ddDAPR and AzddDAPR | | | | |
|---|---|---|---|---|
| Compound | Presence of DCF | Inhibitory dose-50 (μM) | | |
| | | CEM | Molt/4F | MT4 |
| ddDAPR | - | 128 | 220 | > 1000 |
| | + | > 1000 | > 1000 | > 1000 |
| AzddDAPR | - | 68 | 59 | 315 |
| | + | 96 | 87 | 357 |

Table 5

| Kinetic properties of beef intestine adenosine deaminase for 2,6-diaminopurine 2′,3′-dideoxynucleoside analogues | | | |
|---|---|---|---|
| Compound | $K_m$ (μM) | $V_{max}$ (μmole/mg protein/min) | $V_{max}/K_m$ |
| Ado | 29[a] | 287[a] | 9.9[a] |
| ddAdo | 73[a] | 262[a] | 3.6[a] |
| ddDAPR | 29[a] | 8.2[a] | 0.28[a] |
| AzddDAPR | 11 | 40 | 3.56 |
| araDAPR | 83 | 5.9 | 0.07 |
| xylodDAPR | 74 | 3.7 | 0.05 |

[a] Data taken from Balzarini, J. et al (1987c)

## Claims

1. 3$'$-azido-2,6-diaminopurine-2$'$,3$'$-dideoxyriboside.

2. A therapeutic composition for use in the treatment of retroviral diseases including hepatitis B, which comprises as active ingredient 3$'$-azido-2,6-diaminopurine-2$'$,3$'$-dideoxyriboside.

3. A therapeutic composition as claimed in claim 2, comprising said active ingredient in a concentration ranging from 0.1 - 100% by weight.

4. A therapeutic composition as claimed in claim 3, having the form which is selected from the group consisting of powders, suspensions, solutions, sprays, emulsions, unguents and creams.

5. A therapeutic composition for use in the treatment of AIDS or AIDS-related diseases which comprises as active ingredient 3$'$-azido-2,6-diaminopurine-2$'$,3$'$-dideoxyriboside.

6. A therapeutic composition as claimed in claim 5, comprising said active ingredient in a concentration ranging from 0.1 - 100% by weight.

7. A therapeutic composition as claimed in claim 6, having the form which is selected from the group consisting of powders, suspensions, solutions, sprays, emulsions, unguents and creams.

8. A method for the treatment of a retroviral disease which comprises administering of a drug 3$'$-azido-2,6-diaminopurine-2$'$,3$'$-dideoxyriboside to a patient suffering from the retroviral disease, including hepatitis B.

9. A method for the treatment of AIDS or AIDS-related diseases which comprises administering of a drug which is 3$'$-azido-2,6-diaminopurine-2$'$,3$'$-dideoxyriboside to a patient suffering from AIDS or AIDS-related diseases.

10. Use of 3$'$-azido-2,6-diaminopurine-2$'$,3$'$-dideoxyriboside as an active ingredient for preparing a therapeutic composition against a retroviral disease, including hepati tis B.

11. Use of 3$'$-azido-2,6-diaminopurine-2$'$,3$'$-dideoxyriboside as an active ingredient for preparing a therapeutic composition against AIDS or AIDS-related diseases.

EP 0 311 108 A2

# FIG.1

ddDAPR

AzddDAPR

xylodDAPR

araDAPR

FIG.2